# EUROPEAN PATENT SPECIFICATION

(11) **EP 0 841 939 B1**
(45) Date of publication and mention of the grant of the patent: **15.09.2004**
(21) Application number: 96922162.1
(22) Date of filing: 05.07.1996
(51) Int. Cl.: A61K 39/00, C07K 16/18

(54) **THERAPEUTIC AGENTS AND AUTOIMMUNE DISEASES**
MITTELN ZUR BEHANDLUNG VON AUTO-IMMUN KRANKHEITEN
AGENTS THERAPEUTIQUES POUR MALADIES AUTO-IMMUNES

(30) Priority: 05.07.1995 GB 9513733
(43) Date of publication of application: 20.05.1998
(73) Proprietor: UNIVERSITY OF BRISTOL, Clifton Bristol BS8 1TH (GB)
(72) Inventor: WILLIAMS, Neil, Andrew, Axbridge, Somerset BS26 2EF (GB); HIRST, Timothy, Raymond, Clevedon, North Somerset BS21 7RR (GB); NASHAR, Toufic, Osman, University of Bristol, Bristol BS8 1TD (GB)
(74) Representative: Harding, Charles Thomas
(86) International application number: PCT/GB1996/001614
(87) International publication number: WO 1997/002045

(56) References cited:
- WO-A-95/10301
- PROCEEDINGS OF THE NATIONAL ACADEMY OF SCIENCES OF THE UNITED STATES OF AMERICA 93 (1). 1996. 226-230. ISSN: 0027-8424, XP002019422 NASHAR T O ET AL: "Potent immunogenicity of the B subunits of Escherichia coli heat-labile enterotoxin: Receptor binding is essential and induces differential modulation of lymphocyte subsets."
- INTERNATIONAL IMMUNOLOGY 8 (5). 1996. 731-736. ISSN: 0953-8178, XP002019423 NASHAR T O ET AL: "Cross-linking of cell surface ganglioside GM1 induces the selective apoptosis of mature CD8+ T lymphocytes."
- THE JOURNAL OF IMMUNOLOGY, vol. 154, 1995, pages 3611-3617, XP002019424 B. YANKELEVICH ET AL.: "Prevention of Acute Graft-Versus-Host Disease"

## Description

This invention relates to therapeutic agents for use in the treatment of mammalian, particularly human, autoimmune diseases. The invention also relates to therapeutic agents useful in the treatment of human leukaemias of a T cell origin, as so-called "vaccine carriers", and as agents for use in the prevention of human transplantation rejection and graft versus host disease (GVHD).

In an article entitled "Morphologic and Functional Alterations of Mucosal T Cells by Cholera Toxin and its B subunit" by Charles O. Elson et al., The Journal of Immunology, 1995, 154; 1032-1040 it is disclosed that the cholera toxin (Ctx) and the CtxB subunit inhibit CD8⁺ and CD4⁺ T cells.

Reference is also made to the paper entitled "Prevention of Acute Graft-Versus-Host Disease by Treatment with a Novel Immunosuppressant" by B. Yankelevich et al., The Journal of Immunology, 1995, 154: 3611-3617. This identifies CtxB as an agent for use in bone marrow transplantation for the prevention of acute graft-versus-host disease (GVHD).

WO 95/10301 discloses an immunological tolerance- inducing agent comprising a mucosa-binding molecule linked to a specific tolerogen.

As used herein, the term "Ctx" refers to the cholera toxin and "CtxB" to the B subunit of the cholera toxin. In other texts, these may sometimes be identified as "CT" or "Ct" and "CTB" or "CtB" respectively. The term "Etx" herein means the *E. coli* heat labile enterotoxin, and "EtxB" is the B subunit of Etx. In other texts, these may sometimes be identified as "LT" or "Lt" and "LTB" or "LtB" respectively.

The basis for all aspects of the present invention is the finding that EtxB (the pure B-subunit of the E. coli heat labile enterotoxin) binds to GM1-ganglioside receptors which are found on the surfaces of mammalian cells, and that this binding induces differential effects on lymphocyte populations, including a specific depletion of CD8⁺ T cells and an associated activation of B cells. These effects are absent when a mutant EtxB protein lacking GM1 binding activity is employed.

### Autoimmune disease

Autoimmunity is the term used to describe the mechanism by which the body generates an immune response to self-antigens.

In accordance with a first aspect of the invention, there is provided:
the use of an agent in the manufacture of a medicament for use in the treatment or the prevention of an autoimmune disease,
wherein the agent is Etx (E.coli heat labile enterotoxin), EtxB (E.coli heat labile enterotoxin subunit B), Ctx (cholera toxin) and/or CtxB (cholera toxin, subunit B) and wherein, if the agent is to be co-administered with an antigenic determinant, then the agent and antigenic determinant are not so linked to form a single active agent.

Agents for use in accordance with the present invention have been found to modulate lymphocyte populations leading to the induction of apoptosis in CD8⁺ T cells, the enhanced activation of CD4⁺ cells and polyclonal activation of B cells. These events are likely to shift the immune response towards induction of Th2 associated cytokines. Such responses to self or cross-reacting antigens are understood to mediate protection for certain autoimmune diseases.

In a first embodiment of this first aspect of the present invention, the agent is used in a method of treating an autoimmune disease which is in progress. In this embodiment, the agent is administered to a patient with or without co-administration of a self or cross-reacting antigen. Administration of the agent in accordance with this embodiment of the first aspect of the invention modulates the nature of the immune response towards the self-antigen away from the activation of disease-causing inflammation and hence protects against autoimmune disease.

In a second embodiment of this first aspect of the present invention, the agent is used in a method for the "vaccination" of a mammalian subject against an autoimmune disease, in which the agent is co-administered with the self or cross-reacting antigenic determinant (or a combination of different self or cross-reacting antigenic determinants) associated with said disease. In such a manner, the subject's immune response to the self-antigen or cross-reacting antigen is switched away from the activation of pathogenesis, which therefore protects against future autoimmune response to the self-antigen.

In this first aspect of the invention, the therapeutic agent and the self or cross-reacting antigenic determinant are, or may be, co-administered to the subject. By this we mean that the site and time of administration of each of the therapeutic agent and the antigenic determinant are such that the necessary modulation of the immune system is achieved. Thus, whilst the therapeutic agent and the antigenic determinant may be administered at the same moment in time and at the same site, there may be advantages in administering the therapeutic agent at a different time and to a different site from the antigenic determinant.

Whilst single doses of the therapeutic agent and the antigenic determinant may be satisfactory, multiple doses are contemplated within the scope of this aspect of the invention.

Separate administration, in which the therapeutic agent and the antigenic determinant are not linked to form a single active agent is preferred because is enables separate administration of the different moieties.

Specific autoimmune diseases which may be treated by such a medicament are the autoimmune diseases where pathology is associated with cell-mediated immunity, such as rheumatoid arthritis, multiple sclerosis and diabetes.

Also provided is a pharmaceutical composition comprising an agent for use in the treatment of a human autoimmune disease, wherein the agent is Etx (E.coli heat labile enterotoxin), EtxB (E.coli heat labile enterotoxin subunit B), Ctx (cholera toxin) and/or CtxB (cholera toxin subunit B) and wherein, if the agent is to be co-administered with an antigenic determinant, then the agent and antigenic determinant are not so linked to form a single active agent.

The pharmaceutical composition of this aspect of the invention may be formulated to be delivered by a mucosal route, for example as a nasal spray, or parenterally in which the composition is formulated in an injectable form, for delivery by, for example, an intravenous, intramuscular or subcutaneous route.

The pharmaceutical composition may be formulated together with the appropriate self or cross-reacting antigen. Alternatively, a kit may be provided comprising separate compositions for each of the therapeutic agent and the antigenic determinant.

The agents for use in the first aspect of the present invention should preferably be substantially non-toxic, although some degree of toxicity may be tolerated in a severe therapy of this kind.

The first aspect of the present invention relates to the use of Etx or EtxB protein as a therapeutic agent in a medicament for the treatment of a human autoimmune disease. The use of the EtxB protein (which is a pentamer of five identical subunits) for such a treatment represents a preferred embodiment of the present invention.

### T-lymphocyte leukaemias

According to a second aspect of this invention, there is provided:
the use of an agent in the manufacture of a medicament for use in the treatment of human leukaemias or a T cell origin, such as human leukaemias of a CD8 T cell origin wherein the agent is Etx (E.coli heat labile enterotoxin), EtxB (E.coli heat labile enterotoxin subunit B), Ctx (cholera toxin) and/or CtxB (cholera toxin subunit B) and wherein, if the agent is to be co-administered with an antigenic determinant, then the agent and antigenic determinant are not so linked to form a single active agent.

The agents for use in the second aspect of the present invention should preferably be substantially non-toxic, although some degree of toxicity may be tolerated in a severe therapy of this kind.

Also provided is a pharmaceutical composition comprising an agent for use in the treatment of human leukaemias of a T cell origin wherein the agent is Etx (E.coli heat labile enterotoxin), EtxB (E.coli heat labile enterotoxin subunit B), Ctx (cholera toxin) and/or CtxB (cholera toxin subunit B) and wherein, if the agent is to be co-administered with an antigenic determinant, then the agent and antigenic determinant are not so linked to form a single active agent.

The pharmaceutical composition of this aspect of the invention may be formulated to be delivered by a mucosal route, for example as a nasal spray, or parenterally in which the composition is formulated in an injectable form, for delivery by, for example, an intravenous, intramuscular or subcutaneous route.

The second aspect of the present invention relates to the use of Etx or EtxB protein as therapeutic agents in a medicament for the treatment of human T cell leukaemias. The use of the EtxB protein for such a treatment represents a preferred embodiment of the present invention.

### Transplant rejection and GVHD

In accordance with a third aspect of the invention, there is provided:
the use of a therapeutic agent in the manufacture of a medicament for use in the prevention/treatment of transplant rejection or GVHD, wherein the agent is Etx (E.coli heat labile enterotoxin) or EtxB (E.coli heat labile enterotoxin subunit B) and wherein, if the agent is to be co-administered with an antigenic determinant, then the agent and antigenic determinant are not so linked to form a single active agent.

In preferred embodiments of this aspect of the invention, the therapeutic agents described may be used in the manufacture of a medicament for use in the prevention of solid organ transplant rejection, either allogeneic or xenogeneic. They may also be employed in the prevention of acute graft versus host disease (GVHD), for example during bone marrow transplantation procedure.

In embodiments of this aspect of the invention where the patient is treated prior to transplantation, the therapeutic agent would be co-administered with alloantigen or xenoantigen. In embodiments in which the patient is treated after transplantation, the therapeutic agent is employed without co-administration of antigen.

In the embodiment of this aspect of the invention, where the therapeutic agent and allo- or xeno-antigenic determinant are co-administered to the subject, we mean that the site and time of administration of each of the therapeutic agent and the antigenic determinant are such that the necessary modulation of the immune system is achieved. Thus, whilst the therapeutic agent and the antigenic determinant may be administered at the same moment in time and at the same site, there may be advantages in administering the therapeutic agent at a different time and to a different site from the antigenic determinant. Separate administration, in which the therapeutic agent and the antigenic determinant are not so linked is preferred because it enables separate administration of the different moieties.

Whilst single doses of the therapeutic agent and the antigenic determinant may be satisfactory, multiple doses are contemplated within the scope of this aspect of the invention.

In this aspect of the invention, where the agent is being used in the prevention of GVHD, the agent would normally be applied direct to the cells, for example bone marrow cells, to be transplanted.

The agent is preferably substantially non-toxic, although some degree of toxicity may be tolerated in severe therapies of this kind.

Also provided is a pharmaceutical composition comprising an agent for use in the treatment of transplant rejection, wherein the agent is Etx (E.coli heat labile enterotoxin) or EtxB (E.coli heat labile enterotoxin subunit B) and wherein, if the agent is to be co-administered with an antigenic determinant, then the agent and antigenic determinant are not so linked to form a single active agent.

The pharmaceutical composition of this aspect of the invention may be formulated to be delivered by a mucosal route, for example as a nasal spray, or parenterally in which the composition is formulated in an injectable form, for delivery by, for example, an intravenous, intramuscular or subcutaneous route.

The pharmaceutical composition may be formulated together with the appropriate allo- or xeno-antigeneic determinant. Alternatively, a kit may be provided comprising separate compositions for each of the therapeutic agent and the antigenic determinant.

The third aspect of the invention relates to the use of Etx or EtxB protein as a therapeutic agent in a medicament for the treatment of a transplant rejection. The use of the EtxB protein (which is a pentamer of five identical subunits) for such a treatment represents a preferred embodiment of the present invention.

CtxB and EtxB have already been suggested as so-called "vaccine carriers". It has now been discovered that the basis for this effect, in part, is the ability of EtxB to modulate lymphocyte populations (as discussed above) by binding to the GM-1 receptor.

The agent is capable of modulating the immune response when delivered together with an unrelated foreign antigenic determinant. When the agent is delivered parenterally, such immunomodulation is in terms of the immune response being "directed" in a particular desired direction. Where the agent is delivered mucosally with an unrelated antigen, as a so-called "mucosal adjuvant", the agent is capable of facilitating a mucosal immune response to the unrelated antigen. The antigen and agent may be delivered together as separate moieties.

The agent is preferably non-toxic. In addition, where the agent is to be delivered mucosally through the gastrointestinal muscosa, it should be able to remain stable during transit through the gastrointestinal tract; for example, it should be resistant to proteolytic degradation, stable at acid pH and resistant to the detergent effects of bile.

When the therapeutic agent of the invention is a protein, such as the EtxB subunit, it may be produced, for use in all aspects of this invention, by a method in which the gene or genes coding for the specific polypeptide chain (or chains) from which the protein is formed, is inserted into a suitable vector and then used to transfect a suitable host. For example, the gene coding for the polypeptide chain from which the EtxB assemble may be inserted into, for example, plasmid pMMB68, which is then used to transfect host cells, such as Vibrio sp.60. The protein is purified and isolated in a manner known per se. Mutant genes expressing active mutant EtxB protein may then be produced by known methods from the wild type gene.

As previously stated, agents having GM-1 binding activity, such as specifically designed humanised monoclonal antibodies, may be designed and produced as outlined above, by methods which are known in the art.

In all aspects of the invention, the agent having GM1 binding activity may also be capable of cross-linking GM1 receptors. EtxB is one such agent which is capable of cross-linking GM1 receptors by virtue of its pentameric form.

The invention will now be illustrated by reference to the accompanying drawings and the following examples.

### BRIEF DESCRIPTION OF THE DRAWINGS

Figure 1 represents an analysis of physico-chemical properties of EtxB and a mutant form of EtxB, (EtxB(G33D));
Figure 2 illustrates that receptor binding by EtxB is essential for its potent immunogenicity in vivo;
Figure 3 illustrates the kinetics of lymphocyte proliferation following injection of mice with EtxB;
Figure 4 illustrates that EtxB causes increased activation of B cells;
Figure 5 illustrates that EtxB causes increased activation of CD4⁺ T cells and depletion of CD8⁺ cells.
Figure 6 shows the selective depletion of OVA-responsive CD8⁺ T cells by EtxB;
Figure 7 shows that receptor binding by EtxB induces alterations in lymphocyte nuclear morphology characteristic of cells undergoing apoptosis;
Figure 8 shows EtxB receptor-mediated apoptosis of CD8⁺ T cells as measured by cell cycle analysis;
Figures 9a and 9b show the results of experiments conducted to show that GM-1 binding by EtxB inhibits the development of collagen induced arthritis in an animal model;
Figure 10 shows the results of an experiment conducted to illustrate that EtxB but not EtxB(G33D) induces apoptosis in a population of normal human peripheral blood mononuclear cells; and
Figure 11 shows the results of an experiment which shows that cross-linking of GM1 leads to apoptosis in a proportion of murine CTLL cells.

### DETAILED DESCRIPTION OF THE DRAWINGS

### Figure 1

### Analysis of physico-chemical properties of EtxB and EtxB (G33D).

(A) SDS-PAGE analysis of EtxB or EtxB (G33D): 5µg of each protein were analysed under reducing conditions in the presence of β-mercaptoethanol with or without prior heating. Lane 1, wild type EtxB, unheated. Lane 2, EtxB (G33D), unheated. Lane 3; wild type EtxB, heated at 95°C. Lane 4, EtxB (G33D), heated at 95°C. Molecular weight standards (BioRad) are shown on the left-hand side of the panel.
(B) Determination of apparent molecular mass of EtxB and EtxB (G33D) by gel filtration chromatography: standard curve (circles) was generated using, from top to bottom: bovine serum albumin (66 kDa), hen egg albumin (45 kDa), bovine erythrocyte carbonic anhydrase (29 kDa) and horse heart cytochrome c (12.4 kDa); EtxB and EtxB (G33D) eluted with apparent molecular masses of 36kDa and 38kDa respectively; Ve-elution volume of the protein, Vo-void volume of the gel filtration column.
(C) ELISA for comparative binding of EtxB and EtxB (G33D) to ganglioside GM1: plates were coated with GM1, blocked and incubated with 1µg/ml of either EtxB or EtxB (G33D) diluted serially (3 fold) from 1µg/ml.

### Figure 2

### Receptor binding by EtxB is essential for its potent immunogenicity in vivo.

BALB/c mice (4 in each group) were either injected s.c. with EtxB or EtxB (G33D) in PBS or given the proteins orally in bicarbonate buffer. Sera were analysed 10 days following two s.c. injections (A) or 1 week following 3 oral doses (B), and gut secretions were analysed 1 week following 3 oral doses (C). No reaction was detected in samples from control mice (not shown). Results are expressed as mean IgG antibody titre in serum, while IgA in gut secretion is expressed as 'specific activity' as described below.

### Figure 3

### Kinetics of lymphocyte proliferation

Mice were injected i.p. with 30 µg of EtxB (G33D) in complete Freund's adjuvant. MLNs were isolated 10 days later and cells were incubated in the absence of antigen (open square) or in the presence of 80 µg/ml EtxB (filled triangles), EtxB (G33D) (open triangles) or their disassembled monomeric forms of EtxB (filled circles) and EtxB (G33D) (open circles) generated by heating at 95°C. The last 6 hour on each sampling day cells were pulsed with 1µCi of (³H) Thd. Data represents mean cpm and SEM of triplicate wells.

### Figure 4

### EtxB causes increased activation of B cells.

Mice were immunized with EtxB (G33D) in CFA. Cells were isolated from MLN 10 days later and incubated in the presence of 80µg/ml of either EtxB or EtxB (G33D) or a mixture of 40µg/ml of each protein. Cells were labelled with biotinylated anti-CD25 (7D4) and Phycoerythrin (PE) anti-B220 (Ra3-6D2). Streptavidin FITC was used as a secondary antibody conjugate. Controls for the antibodies were also included (not shown). Dual flow cytometric analysis was performed on day 4 of proliferation.

### Figure 5

### EtxB causes increased activation of CD4+T cells and depletion of CD8*T cells.

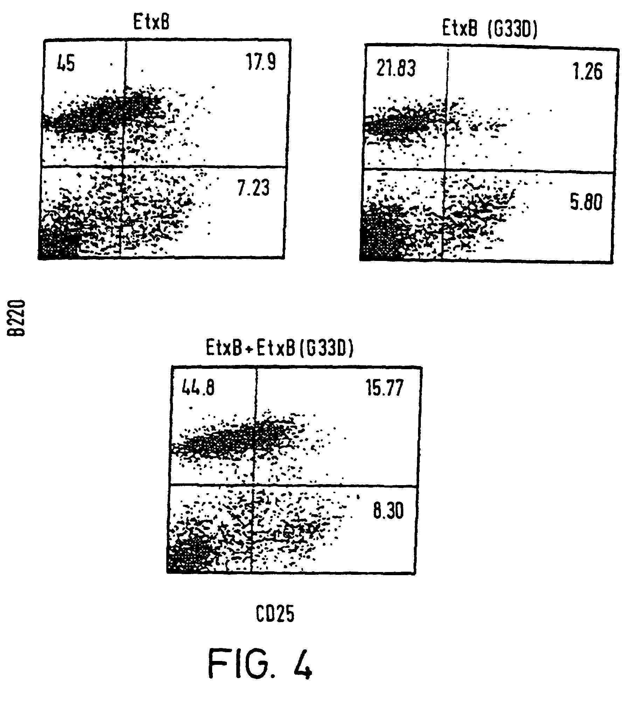

The immunization procedure, cell isolation and the in vitro challenge are as described in the legend of Fig. 4. To detect CD25, biotinylated anti-CD25 (7D4) and Streptavidin FITC were used. To detect CD4 and CD8, FITC labelled anti-CD4 (RNRM4-5) and FITC labelled anti-CD8α (53-6.7) were used. Appropriate controls for the antibodies were included (not shown).

### Figure 6

### Selective depletion of OVA-responsive CD8⁺ T cells by EtxB

Cultures of cells from MLN taken from OVA-primed mice were established for 5 days, in the absence of antigen or in the presence of OVA + EtxB, OVA + EtxB(G33D) or OVA alone at 100 µg OVA and 40 µg/ml each of EtxB or EtxB(G33D) or 100 µg OVA alone. Cells were labelled with the following rat antibodies: FITC-anti-CD4 or FITC-anti-CD8α and both with biotin-anti-CD25 (IL-2Rα) followed by Streptavidin-phycoerythrin. Non-stained cells or cells stained with the second antibody alone were also included as controls. Cells were analysed by FACS (Becton Dickinson). The higher increase in the proportion of total cells which are CD25+ in cultures containing EtxB compared with other treatments is due to the presence of higher proportion of B cells expressing this marker (not shown). The scale of fluorescence intensity is log.

### Figure 7

### Receptor binding by EtxB induces alterations in lymphocyte nuclear morphology characteristic of cells undergoing apoptosis.

MLNC comprising >90% CD3⁺ T cells and depleted of macrophages were incubated for 18 h with either 80 µg/ml EtxB or with 80 µg/ml EtxB(G33D) and stained with acridine orange. Cells were examined under conventional or confocal fluorescence microscopy (Leica TCS 4D). A representative microscopic field (x 540) for each treatment is shown [EtxB, left hand panel; EtxB (G33D), right hand panel]. Cells which were incubated in the absence of antigen gave similar results to those treated with EtxB(G33D) (not shown).

### Figure 8

### EtxB receptor-mediated apoptosis of CD8⁺ T cells as measured by cell cycle analysis.

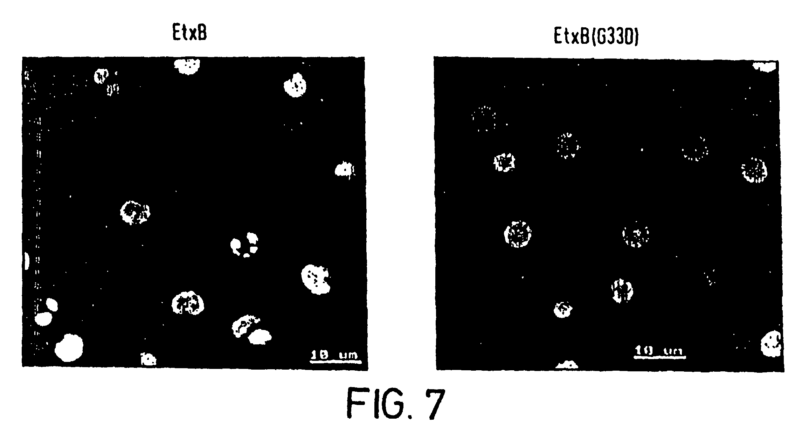

The proportion of CD4⁺ and CD8⁺ SPLTC in the sub-G₀/G₁ stage of the cell cycle was determined by flow cytometric analysis of the DNA content following staining with propidium iodide. SPLTC were isolated from the spleen by negative selection as described above. The cells were treated for 18 h with: (a) no antigen, (b) 80 µg/ml EtxB(G33D) or (c) 80 µg/ml EtxB and then stained with FITC-rat anti-CD4 or FITC-rat anti-CD8α. The cells were subsequently stained with propidium iodide. The proportion of cells co-stained with propidium iodide was determined by gating on cells stained with either anti-CD4 or anti-CD8 antibodies. This experiment has been carried out on cells, results of which are also reported in Figure 7 and Table 3.

### Examples

### Example 1

This example illustrates the requirement for GM-1 binding to induce differential effects on lymphocyte populations.

### Materials and Methods

### Generation of a receptor-binding mutant of EtxB

A Gly-33 to Asp substitution was introduced into the receptor binding site of human EtxB using plasmid pTRH29, a derivative of the phagemid vector pBluescript IIKS+, that contains the genes for the A- and B-subunits of Etx (Yu, J., Webb, H. & Hirst, T.R. (1992), Molec. Microbiol. 6, 1949-1958). Mutagenesis was performed with an in vitro oligonucleotide-directed mutagenesis kit (Amersham International) using single-stranded pTRH29 as a template and a synthetic oligonucleotide (5'-TCTCTTTTATCTGCCATCG-3') (from the Microanalytical Facility, IAPGR, Cambridge Research Station, UK) as the mutagenic primer. The correct Gly to Asp substitution was confirmed by dideoxy sequencing using Sequenase II (United States Biochemical Corp.) and the resultant plasmid was designated pTRH56. The mutant *etxB* gene from pTRH56 was excised, using *Eco*RI and *Spe*I restriction enzymes, and inserted into pMMB68 (Sandkvist, M., Hirst, T.R. & Bagdasarian, M. (1987) J. Bacteriol. 169, 4570-4576) to yield a broad host range expression vector, pTRH64 expressing EtxB(G33D). Antigens

Wild-type EtxB and EtxB(G33D) were purified from culture supernatants of *Vibrio* sp.60 (pMMB68) and *Vibrio* sp.60 (pTRH64), respectively, using a modification of the method reported by Amin and Hirst (Amin, T., & Hirst, T.R. (1994) Prot. Express. and Purif. 5, 198-204). Briefly, proteins were purified by diafiltration and hydrophobic interaction chromatography and concentrated by anion-exchange chromatography. The protein solutions were desalted on a PD10 column (Pharmacia, UK) equilibrated with phosphate buffered saline (PBS; 10mM sodium phosphate, 150 mM NaCl, pH7.4) and stored at -30°C.

The purity of EtxB and EtxB(G33D) were confirmed by SDS polyacrylamide gel electrophoresis. The molecular mass of the individual monomers were confirmed by laser desorption mass spectrometry (Protein Science Facility, University of Kent).

Apparent molecular masses of EtxB and EtxB(G33D) were determined by gel filtration chromatography using a SMART system (Pharmacia). Proteins were eluted from a Superdex 75 PC 3.2/30 column in PBS, pH7.5.

Irreversible denaturation of B subunit pentamers, for use in lymphocyte proliferation assays (see below), was achieved by heating the proteins at 95°C for 5 min. Animals, sample collection and immunization protocols

BALB/c mice (H-2^{d}; high responder to EtxB) of 7-12 weeks of age were purchased from Charles River Laboratories and maintained at the University of Kent animal house. Antibody responses to EtxB or EtxB(G33D) were measured after s.c. injection of mice with 30µg of protein in PBS, followed by boosting 10 days later. Another group of mice were given the same protein dose orally in sodium bicarbonate (50µg/ml) on 3 occasions, and at one week intervals. Control mice were given PBS. Blood was collected 10 days following the last s.c. injection or one week following the last oral feeding. Gut secretions from live mice were isolated in a protease inhibitor solution as previously described (Elson, C.O., Ealding, W. & Lefkowitz, J. (1984) J Immunol. Meth. 67, 101- 108), one week following the last feeding. Samples were then sonicated and clarified by centrifugation (13,226 x g, 10 min, at 4°C).

For the proliferative assays, mice were injected i.p. with 30µg of EtxB or EtxB(G33D) in complete Freund's adjuvant (CFA) and the mesenteric lymph nodes isolated 10 days later. Control unimmunized mice were also included and their lymph nodes isolated in a similar manner.

### Enzyme Linked Immunosorbent Assays (ELISAs)

Binding of EtxB or EtxB(G33D) to GM1 was examined by a GM1-ELISA (Amin, T., & Hirst, T.R. (1994) Prot. Express. and Purif. 5, 198-204).

Sera and gut secretions were examined for the presence of anti-B subunit IgG and IgA antibodies by ELISA's in which samples were applied to microtitre plates (Immulon I, Dynateck, USA) coated with 5µg/ml of either EtxB or EtxB (G33D) in PBS. Anti-B subunits IgA antibodies in gut secretion supernatants were extrapolated from a standard curve made by coating 2 rows of wells on each plate with 1µg/ml rabbit anti-mouse IgA (α chain specific; Zymed Lab, USA) in PBS followed by addition of 1µg/ml of mouse myeloma IgA (MOPC 315, Sigma, USA). To measure total IgA, wells were coated with rabbit anti-mouse IgA followed by addition of gut secretion supernatants. All samples were serially diluted. Goat anti-mouse IgG (Fc fragment specific; Jackson Lab., USA) or goat anti-mouse IgA (a chain specific; Sigma) peroxidase conjugate were diluted and added to all wells. The anti-B subunit IgG titer, giving an A₄₅₀ₙₘ ≥ 0.2, was determined. The IgA anti-B subunit response for each of EtxB and EtxB (G33D) in gut secretions was calculated as "IgA specific activity" [mean IgA anti-B subunit (µg/ml)/total IgA (µg/ml)].

An ELISA method for measuring cytokine levels of IL-2, IL-4, IL-5, IL- 10 and IFN-γ was used, as described previously (Harper, H.M., PhD thesis, Univeristy of Bristol (1995)). Briefly, microtiter plates were coated with rat antibodies to mouse IL-2, IL-4, IL-5, IL-10 and IFN-γ. Plates were blocked with 2% (w/v) bovine serum albumin. Supernatants from culture medium were added to wells and diluted down. One row on each plate for each cytokine contained a standard amount of recombinant cytokines. Plates were then incubated with 0.5µg/ml of biotinylated anti-cytokine monoclonal antibodies followed by addition of avidine-peroxidase and 3,3',5,5'-Tetramethylbenzidene (TMB) substrate and read at A₄₀₅ₙₘ.

### Lymphocyte proliferation assay

Mice were sacrificed by cervical dislocation, mesenteric lymph nodes were excised aseptically and minced through a stainless steel mesh into Hank's balanced salt solution (HBSS) (Flow Laboratories, Irvine, Renfrewshire, UK.). Cells were washed by centrifugation (500 x g, 10 min, 4°C) in HBSS and resuspended in modified Eagle's medium (Flow) to which 20mM Hepes (Flow), 100 IU Penicillin, 100µg/ml Streptomycin, 4mM L-glutamine (Flow) and 2-mercaptoethanol had been added (complete medium). Fresh autologous normal mouse serum from unimmunized mice was added to a final concentration of 0.5% (v/v). Cultures contained 2 x 10⁶ viable cells/ml in either 2 ml volumes in 24-well plates or 8 ml volumes in 25 cm³ flasks (Nunc A/S, Roskide, Denmark) and were established in the presence and absence of antigens as indicated in the figures legend. Cultures were incubated at 37°C in a humidified atmosphere of 5% CO₂ and 95% air for 6 days. At desired timepoints, 0.1 ml samples were removed from the cultures and transferred to 96 well U-bottomed plates (Nunc) and pulsed with 1µCi/well of [³H]-Thd (Amersham, U.K) for 6 h before harvesting (Mach III harvesting 96 Tomtec, Orange, Conn. USA) and counting by standard liquid scintillation 1450 Micro β plus, LKB-Wallac, Turku, Finland). Similarly, 0.5 ml of supernatant was sampled from cultures for cytokine analysis. Cells were pelleted and the supernatants stored at - 68°C until analysed.

### Phenotypic analysis of cultured cells

Cultured cells harvested on day 4 of culture were washed and viable cells recovered at the interface of a HBSS/18% metrizamide (Nyegaard and Co., Oslo, Norway) gradient following centrifugation at 500 xg for 15 min at 20°C. Cells were washed twice and resuspended in HBSS containing 0.2 % sodium azide (Sigma) and 10% normal rat serum. The following rat antibodies (Pharmingen, San Diego, USA) were used: fluorescein isothiocyanate (FITC) labelled anti-CD4 (RNRM4-5), FITC labelled anti-CD8 (53-6.7), biotin-labelled anti-CD25 (7D4) and Phycoerythrin (PE) labelled anti-B220 (RA3-6D2). Additionally, for the biotin-labelled antibodies Streptavidin-PE or Streptavidin-FITC (Serotech, UK) were used. All antibodies were diluted in HBSS containing azide and used at predetermined concentrations. 200µl of 2 x 10⁶ cells and 200µl of each the antibodies were mixed and incubated on ice for 30 min. When Streptavidin -PE or FITC secondary antibodies were required cells were incubated with these antibodies for additional 30 min. Appropriate controls for FITC and PE antibodies were also included. Cells were washed with HBSS and then analysed by 2 flow cytometery (Becton Dickinson).

### Results

### Generation and characterization of a receptor binding mutant of EtxB

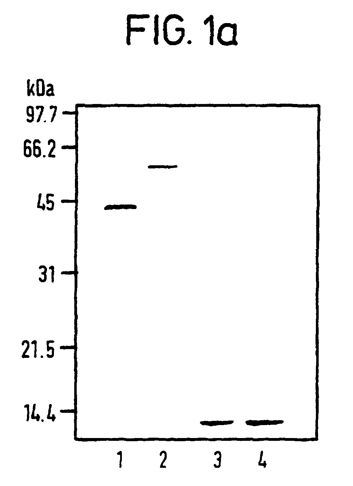

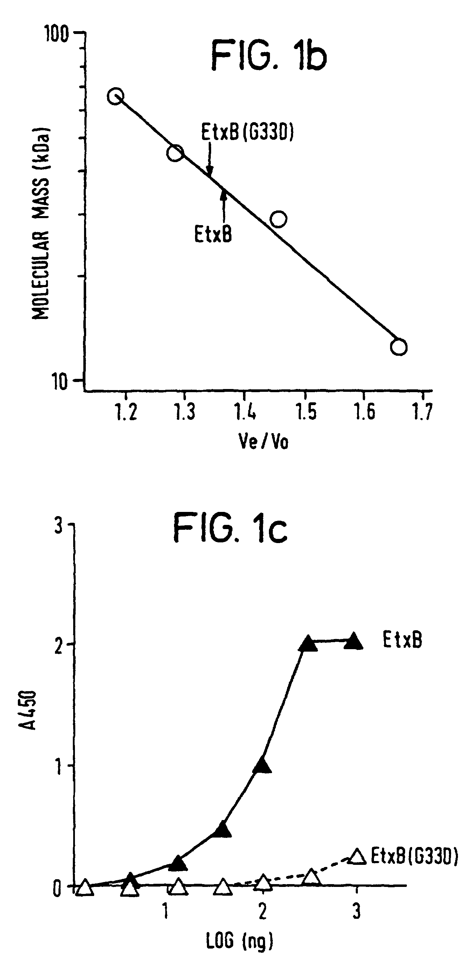

A Gly to Asp substitution was introduced into the B subunit of *E. coli* heat-labile enterotoxin by oligonulceotide-directed mutagenesis of EtxB, in order to generate a mutant B subunit defective in receptor recognition. The mutant protein, designated EtxB(G33D), and wild type EtxB were purified to homogeneity (see Materials and Methods). The molecular mass of purified EtxB and EtxB(G33D) were determined by laser desorption mass spectrometry. Masses were within 20 Da of the theoretical masses of 11702 and 11760 Da for monomeric EtxB and EtxB (G33D), respectively. When analysed by SDS-PAGE without prior heating, both wild-type EtxB and EtxB(G33D) migrated as discrete stable oligomers, with apparent molecular weights of 42 kDa and 56 kDa (Fig.1A, lane 1 and lane 2, respectively). The observed electrophoretic mobility and SDS-stability of EtxB is a characteristic property of the B subunit pentamer (see Sandkvist, M., Hirst, T.R. & Bagdasarian, M. (1987) J. Bacteriol. 169, 4570-4576). The slower electrophoretic mobility of oligomeric EtxB(G33D) is not due to a difference in the number of constituent B subunit monomers, since both pentameric EtxB and EtxB(G33D) exhibited similar retention times when analysed by high resolution gel filtration chromatography. Thus, the discrepancy in the electrophoretic mobility of the EtxB(G33D) oligomer with respect to wild-type EtxB, is likely to be due to the introduced negatively charged Asp residue causing a reduction in SDS binding and a subsequent slower migration.

EtxB and EtxB(G33D) were also compared for their stability in low pH buffers, resistance to 1.0 mg/ml of either trypsin or proteinase K, and relative reactivity towards a panel of anti-B subunit monoclonal and polyclonal antibodies. In each of these tests EtxB(G33D) exhibited identical properties to wild-type EtxB. It is therefore concluded that a Gly to Asp substitution at residue 33 in EtxB does not alter the oligomeric configuration, SDS, pH or protease stability, or antibody reactivity compared with wild-type EtxB.

The ability of EtxB(G33D) to bind to its receptor GM1, was evaluated using a GM1-ELISA (Fig. 1C). This showed a highly significant reduction in the ability of the mutant to bind GM1 compared with the wild type protein (> 99% reduction in the A₄₅₀ₙₘ reading). Furthermore, in contrast to wild type EtxB, EtxB(G33D) failed to bind to CHO cells when examined by immunofluorescence. It is concluded that EtxB(G33D) is defective in its capacity to bind GM1 ganglioside, *in vitro* and *in situ*.

### The potent immunogenicity of EtxB in vivo is dependent on receptor binding

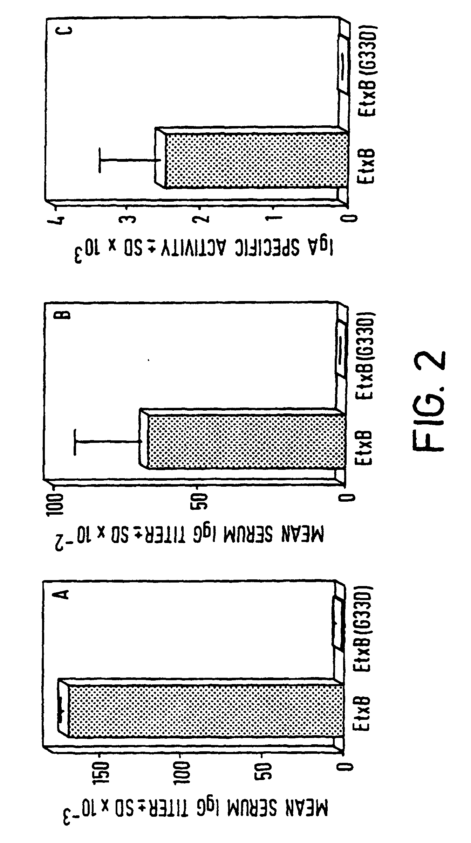

The importance of receptor binding in the immunogenicity of EtxB was evaluated in mice following either oral delivery or s.c. injection of EtxB or EtxB(G33D) in PBS. Oral delivery of EtxB resulted in detection of a high IgG antibody titer in serum and IgA antibody activity in gut secretions (Fig. 2). In contrast, a similar regime of oral immunization with EtxB(G33D) failed to generate any detectable antibody activity. EtxB(G33D) did induce a serum antibody response following s.c. injection, although the response was considerably lower in comparison to the antibody response to wild type EtxB, with > 160 fold reduction in the mean antibody titer, 1050 versus 171000, respectively. It is concluded that receptor binding by EtxB is essential for its potent immunogenicity *in vivo.*

### Receptor binding does not influence the extent of lymphocyte proliferation in the presence of EtxB or EtxB(G33D)

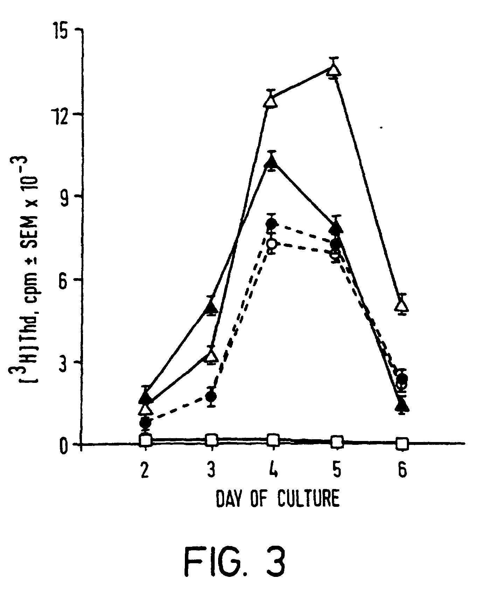

The effect of EtxB or EtxB(G33D) on lymphocyte proliferation *in vitro* was examined. Lymphocytes were isolated from the popliteal and mesenteric lymph nodes (MLN) of mice immunized with either EtxB or EtxB(G33D) and stimulated *in vitro* with either protein, or with a heat-denatured preparation of EtxB or EtxB(G33D). The proliferative response of lymphocytes derived from the popliteal or MLN was similar. In each case, proliferation to each of the protein preparations increased with increasing B subunit concentration. A representative set of data from an experiment using MLN is shown in Table 1. The magnitude of the response to wild type and mutant pentamers was comparable as was that in the presence of heat-denatured wild type and mutant monomers. Figure 3 shows the kinetics of the proliferative responses obtained in the presence of 80 µg/ml of each of the protein preparations. Reactivity was dependent on the presence of antigen, and followed a similar pattern in the presence of each protein. Reactivity was evident on day 3 of culture with incorporation of [³H]-Thd reaching a peak on day 4 and waining thereafter. The minor differences in the timing of peak responses apparent in Figure 3 were not observed in repeat experiments, showing that the anamnestic characteristics of the responses to the EtxB and EtxB(G33D) are comparable. It is concluded that the level of stimulation in the presence of the native proteins is not likely to be influenced by receptor binding or the introduced mutation.

### Toxin receptor binding causes immunomodulation of B cells and T cell subsets

To examine if receptor binding by EtxB exerts any effect on the populations of lymphoid cells *in vitro*, lymphocytes were isolated from the MLN of mice primed i.p. with EtxB(G33D) and then stimulated with either EtxB or EtxB(G33D) or a mixture of both. Additionally, a parallel experiment using MLN-derived lymphocytes from mice injected with EtxB was undertaken and resulted in essentially identical findings to those obtained from EtxB(G33D) primed mice.

### (i) EtxB causes increased activation of B cells

The effect of EtxB on B cells were examined by expression of the activation marker CD25 (IL-2Rα) in association with the B cell marker B220 (CD45R). As shown in Fig.4 the number of B cells in cultures stimulated with EtxB was 62.9% of total cells, of which a high proportion (28.4%) expressed the cell activation marker CD25. In contrast, the proportion of B cells after stimulation in the presence of EtxB(G33D) was less than half of that of the wild type (22.26%) and fewer were activated (5.6%). To establish whether the effects exerted by EtxB were dominant, cells were incubated in the presence of an equimolar concentration of EtxB and EtxB(G33D). The flow cytometric data was similar to that obtained following stimulation in the presence of wild type EtxB alone (with 60.6% B cells, of which 26% were activated). It is concluded that the receptor binding property of EtxB mediates an increased activation of B cells *in vitro*.

### (ii) EtxB causes increased activation of CD4⁺ T cells and complete depletion of CD8⁺ T cells.

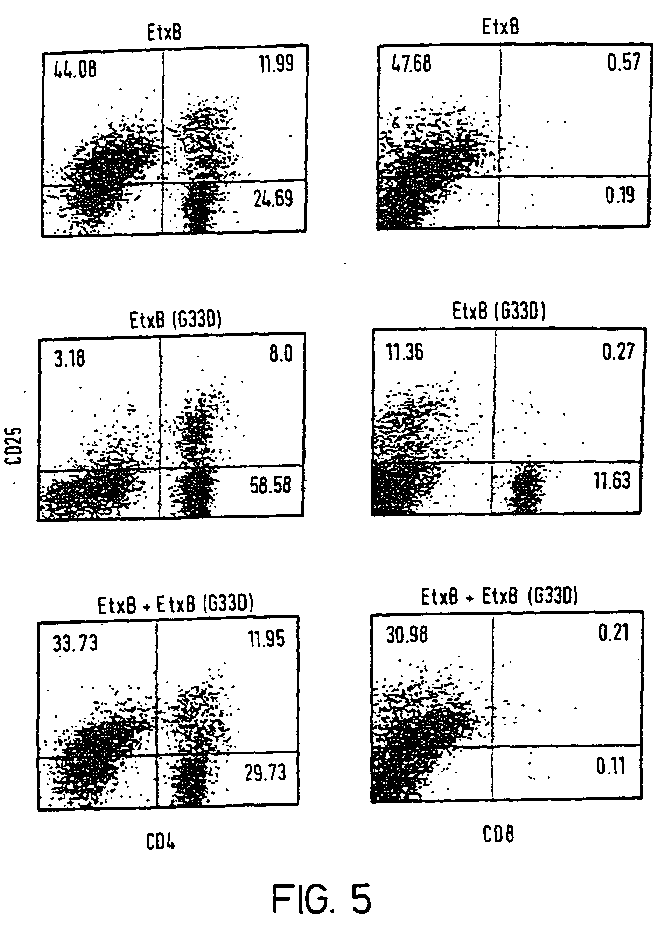

To examine the influence of B subunit receptor binding on T cells, lymphocytes were labelled with antibodies to CD4 or to CD8 in association with antibodies to CD25 (Fig.5). Additionally, some cells were separately labelled with antibodies to the CD3 marker (not shown). The proportion of T cells expressing the CD4 marker when stimulated in the presence of EtxB was 36.7%, of which a high proportion (32.7%) were activated. In contrast, no detectable CD8⁺ T cells were present in the culture containing EtxB.

By comparison, both CD4⁺ and CD8⁺ T cells were present in the culture stimulated in the presence of EtxB(G33D). Such cultures contained a large proportion of CD4⁺ T cells (66.6%), but only 12% of these were activated. The proportion of CD8⁺ T cells detected in the presence of EtxB(G33D) was 11.7% of the total number of cells, but very few of these were activated which is indicated by the absence of the CD25 marker. Additionally, in the presence of a mixture consisting of an equimolar concentration of EtxB and EtxB(G33D) the pattern of responding cells was similar to that in the presence of wild type EtxB alone; with 41.68% CD4⁺ T cells (of which 28.6% were CD25+) and no detectable CD8⁺ T cells (Fig.5). In all these analyses, the proportion of cells staining with CD3 was approximately equal to the sum of those expressing CD4 and CD8 markers. These data demonstrate that the increase in activation of B and CD4⁺ T cells and the selective depletion of CD8⁺ T cells are mediated by toxin receptor occupancy.

### Production of cytokines

To assess whether the effect of EtxB on lymphocyte populations could be dependent on a change in cytokine production, cell cultures were incubated with either EtxB or EtxB(G33D) and supernatants removed on days 2, 3, 4, 5 and 6 for analysis. The results from samples collected on day 5 are shown in Table 2 when the maximum concentration of cytokines was detected. Both IFN-γ and IL-2 were detected in the supernatants from cultures stimulated in the presence of EtxB or EtxB(G33D), although the relative levels of these cytokines varied. The medium from cells incubated with wild type EtxB, contained a 3-fold higher concentration of IL-2 and a 1.5 fold lower level of IFN-γ compared with supernatants from cultures stimulated in the presence of EtxB(G33D). Despite the finding that other proliferating T cell cultures responding to other antigens yielded high levels of IL-4, IL-5 and IL-10 none of these cytokines were detected in cultures stimulated with EtxB or EtxB(G33D). The increase in the level of IL-2 and decrease in the level of IFN-γ following stimulation with EtxB, compared with EtxB(G33D), most likely reflects the activation status of B and CD4⁺ T cells. Nonetheless, the results indicate that the profound effect of wild type EtxB on the
CD8⁺ T cell population is unlikely to be mediated by a major shift in the cytokine profile, as a consequence of receptor occupancy.

### Discussion

These investigations show that the introduction of a single point mutation (G33D) in the receptor binding site of EtxB caused a significant loss in the ability to bind GM1. Importantly, the mutant EtxB(G33D), exhibited identical physico-chemical properties to the wild type EtxB with respect to conformation, as revealed by gel chromatography, stability in SDS, acid and proteases. When the specific antibody responses were measured following immunization with either EtxB or EtxB(G33D), dramatic differences were noted. Subcutaneous injection with EtxB(G33D) in mice resulted in a highly significant drop in the antibody titer compared with wild type (ca >160 folds) while no antibody response was detected following oral administration. It is possible that these differences result from the disruption of a dominant epitope involved either in the recognition of the molecule by antibody, or the stimulation of effective T cell help for antibody production. However, it is noteworthy that the Gly to Asp substitution had no effect on the recognition of the B subunit by a panel of specific polyclonal and monoclonal antibodies. Further, the proliferative responses obtained when EtxB or EtxB(G33D) were added to cultures were comparable regardless of which of the proteins were used for in vivo priming; demonstrating that the T cell reactivity was not specific to either molecule. It is therefore concluded that receptor binding by EtxB is essential for its potent immunogenicity *in vivo.*

The importance of receptor binding in the potent immunogenicity of EtxB may be explained in a number of ways. Firstly, binding of the B subunit of Etx and Ctx to GM1 may increase the efficiency of uptake of these proteins, raising the local protein concentration available to the immune system. Other classes of proteins which are able to bind mucosal surfaces are found to be effective immunogens (De Aizpura, H.J. & Russell-Jones, G.J. (1988) J. Exp. Med. 167, 440- 451). The observed differences in the immunogenicity of EtxB and its mutant following oral administration may indeed be due to efficient uptake of EtxB from the lumen of the gut. However, the dramatic differences noted after parenteral immunization (where antigen is delivered locally at high concentration) are suggestive of other effects. For example, binding of EtxB to GM1 may affect the efficiency of antigen presenting cell activity. Such binding could cause activation of class II-bearing cells, particularly with respect to their expression of essential co-stimulatory molecules, such as B7, which is associated with their acquiring enhanced antigen presenting activity (Jenkins, M.K. & Johnson, J.G. (1993) Curr. Opin. Immunol. 5, 361-367). Alteratively, receptor binding may have direct effects on sub-populations of lymphocytes. A number of observations from this study provide strong evidence that this is indeed the case.

The *in vitro* studies demonstrated that EtxB was able to induce the proliferation of primed lymph node cells. This property was not dependent on receptor binding, since responses with similar anamnestic characteristics were obtained using either wild type EtxB, EtxB(G33D) or heat-denatured monomeric forms of these proteins which cannot bind GM1. These observations are interesting in themselves since it has been widely reported that commercial preparations of Ctx and CtxB or purified recombinant CtxB are strongly inhibitory of lymphocyte proliferation *in vitro*. The apparent discrepancy may have arisen from the fact that previous experiments had been conducted on purified lymphocytes and had largely used mitogen stimulated lymphocyte cultures (which are not clonally restricted responses), where a different mechanism may be involved. Consistent with this was our observation that the proliferation of Con A-stimulated lymphocytes was indeed inhibited by EtxB. However, the analyses of cell populations in cultures of primed lymph node cells stimulated with either EtxB or EtxB(G33D) revealed important differences with respect to B cells as well as CD4 and CD8-bearing T-cells.

B cells were detected after 4 days of culture in the presence of either EtxB or EtxB(G33D). However, by comparison with EtxB(G33D), the relative proportion of B cells present in cultures with EtxB was increased by approximately 100%. This increase was associated with the expression of CD25 on a very high proportion of the B cells. In the experiment shown, the responding lymphocytes were primed with EtxB(G33D) *in vivo*. Similar experiments with cells from EtxB immunized mice revealed comparable results. Thus, irrespective of any *in vivo* effects associated with receptor binding, cultures in the presence of EtxB contained a larger proportion of B cells compared with those stimulated with EtxB(G33D). These effects on B cells also appear not to be dependent, at least in part, on regulation by T cells, *in vitro*, as the results do not suggest a major shift in the profile of the detected cytokines. Therefore, *in vitro*, receptor binding by EtxB appears to be associated with a direct effect on B cells, resulting in proportional expansion of this population as well as their activation. It is also noteworthy that CtxB has been shown to increase expression of MHC class II on virgin B cells, a property which was not exhibited by a GM1 binding mutant CtxB (G33E) (Francis, M.L., Ryan, J., Jobling, M.G., Holmes R.K., Moss, J, & Mond J.J. (1992) J. Immmunol. 148, 1999-2005). The results in these experiments suggest the presence of direct mitogenic effects by EtxB on antigen-primed B cells and demonstrate that such effects are mediated by receptor binding.

In addition to the effects of EtxB on B cells in culture, flow cytometric analyses reveal that this toxoid caused the complete depletion of any detectable CD8⁺ cells. Once again, this effect was shown to be dependent on receptor binding, since this population of T cells were not depleted in cultures containing EtxB(G33D). Further, complete depletion of CD8⁺ cells in cultures containing EtxB was observed, from mice immunized with wild type EtxB. There are three possible mechanisms by which such an effect may be mediated. 1) It is known that binding of Ctx or CtxB to GM1 on rat MLN cells induces patch and cap formation (Craig S.W. and Cuatrecasas P., (1975) Proc. Natl. Acad. Sci. USA, Vol.72, pages 3844-3848). It is possible that in this process EtxB-GM1 complexes and other molecules, including CD8, are internalized. Such a process would prevent flow cytometric detection of these cells using CD8 as a marker, and may result in their death due to the associated loss of the surface TCR complex. Although the latter may account for the absence of CD8⁺ T cells in the culture, others found no loss of the TCR complex from the surface of human Jarkat T cell line when CtxB was used (Imboden, J.B., Shoback, D. M., Pattison, G, & Stobo, J.D. (1986) Proc. Natl. Acad. Sci. USA 83, 5673-5677). Absence of effects as a result of capping is supported by the finding that CD3 and CD4 markers were not affected. 2) An alterative mechanism would involve effects exerted by cytokines in culture. In this study, both IL-2 and IFN-γ were detected. The results, however do not suggest a major shift in the cytokine profile which would explain such a dramatic effect on CD8⁺ T cells. 3) Absence of CD8⁺ T cells may be due to active induction of apoptosis. Death of lymphocytes by apoptosis may involve capping as described above, or could be mediated in the absence of capping by effects on the signalling events in the cell. Activation-induced programmed death is dependent on Ca²⁺ and involves phosphatases and kinases. Binding of CtxB to lymphocytes has been shown to inhibit protein kinase C-dependent proliferation and induced a pronounced increase in intra-cellular Ca²⁺, events which were not associated with an increase in CAMP level. The ability of EtxB to deplete CD8⁺, but not CD4⁺ T cells could be due to differential effects of signals associated with the CD4/CD8-TCR complex, resulting from crosslinking GM1 on the surface of these subset of lymphocytes. This could be as a result of differential binding of the toxoid on the membrane as reported for CtxB or alternatively to the differential signalling mechanisms in CD4⁺ and CD8⁺ T cells.

In the complete absence of detectable CD8⁺ T cells, EtxB increased the proportion of CD4⁺ T cells which were activated, by comparison with the receptor binding mutant. The essential requirement for CD4⁺ T cells in response to Ctx has been demonstrated *in vivo*. The reason for the increased activation of this T cell subset is, however, unclear. It is noteworthy that CtxB has been shown to stimulate DNA synthesis and cell division in quiescent non-transformed mouse 3T3 cells. A selective mitogenic effect on CD4⁺ T cells was also found in the presence of plant lectins which bind to Galß-1-3-3GalNAc, the same component that EtxB binds to in GM1. The possibility can not be ruled out that EtxB mediates a GM1-binding dependent direct effect on CD4⁺ T cells, causing their activation. However, it is also possible that the increased activation of CD4⁺ T cells in cultures containing EtxB is a consequence of those changes to the B cell and CD8⁺ T cells populations described. B cell activation is known to be associated with an enhancement of their competency as antigen presenting cells for CD4⁺ T cells. Further, CD8⁺ T cells are widely associated with a regulatory role in immune reactivity both *in vivo* and *in vitro*. Their removal from T cell proliferative cultures has been associated with prolonged and enhanced levels of CD4⁺ T cell division.

Taken together, the potent immunogenicity of EtxB *in vivo,* as shown in this study, can be suggested to occur as a result of its ability to increase activation of B cells exerted by growth regulating effects following binding to GM1. Activation of CD4⁺ T cells and the ability of EtxB to increase production of IL-2 in culture in vitro may provide the necessary signal for further expansion of B cell clones. Depletion of CD8⁺ T cells by EtxB *in vitro* in this study may also provide another mechanism of immunopotentiation *in vivo* following systemic or oral delivery, particularly in the light of the involvement of this subset of cells in suppression of the immune response and in oral tolerance. In this regard, both Ctx and Etx have been shown to abrogate oral tolerance to cofed soluble proteins and other studies implicated Ctx and CtxB depletion effects on intra-egithelial lymphocytes in the gut or in the dome of Peyer's patch to explain this mechanism. CtxB-inhibitory effects on CD8⁺T cells *in vitro* has also been shown to prevent graft versus host reaction.

In conclusion, it has been demonstrated that the presence of potent immunomodulatory effects by EtxB on the antibody response *in vivo*, and on populations of lymphocytes ir. vitro. Furthermore, it has been demonstrated that these effects are mediated by receptor binding. Our findings are also pertinent to an understanding of the ability of Etx and Ctx to act as potent adjuvants and as potential protein carriers for other antigens and suggest that such properties rely on the capacity of these toxoids to bind ganglioside receptors on the surface of lymphoid cells.

### Example 2

This example illustrates that the effects on CD8 cells are irrespective of antigen recognition and are mediated by apoptosis.

Recombinant preparations of EtxB and EtxB(G33D) were prepared as in Example 1. Both proteins were well characterised with respect to binding to GM1, binding to a panel of monoclonal and polyclonal antibodies and various other physico-chemical properties. Ovalbumin (OVA) was purchased from Sigma (Poole, UK). Mesenteric lymph nodes (MLN) were isolated from BALB/c mice [high responder strain to EtxB (Nashar, T.O. and Hirst, T.R. 1995. Immunoregulatory role of H-2 and intra-H-2 alleles on antibody responses to recombinant preparations of B-subunits of *Escherichia coli* heat-labile enterotoxin (rEtxB) and cholera toxin (rCtxB). *Vaccine* 13:803.)] 8-10 weeks old. Mice were injected i.p. with 200µg of OVA (Sigma) emulsified in incomplete Freund's adjuvant (Sigma). MLN were removed 10 days after injection, minced through a stainless steel mesh into HBSS (Flow, Irvine, UK). The recovered cells were washed in HBSS by centrifugation (500 g, 10 min, 4°C) and resuspended in modified Eagle's medium (Flow) containing 20 mM HEPES, 100 IU penicillin, 100 µg/ml streptomycin, 4 mM L-glutamine and 5 x 10⁻⁵ M 2-mercaptoethanol (complete medium) to which 0.5% (v/v) of fresh autologous mouse serum was added. Cultures contained 2x10⁶ viable cells/ml in 2 ml volumes in 24-well plates (Nunc, Roskide, Denmark) and were established in the presence of 100 µg/ml OVA (dialysed extensively in complete medium), either alone or with 40 µg/ml of EtxB or EtxB(G33D). Cultures were incubated at 37°C in 5% CO₂ and 95% air for 5 days. At desired time points, 0.1 ml samples were removed from the cultures and transferred to 96 well U-bottomed plates (Nunc) and pulsed with 1 µCi/well of [³H]thymidine (Amersham, UK) for 6 h before harvesting (Mach III harvesting 96; Tomtec, Orange, CT) and counting by standard liquid scintillation (1450 Micro b plus; LKB-Wallac, Turku, Finland). For flow cytometric analysis (Becton Dickinson, Erenbodegem-Aalst, Belgium) of T cells, cells were stained with the following rat antibodies (PharMingen, Cambridge, UK): FITC labelled anti-CD4 (RNRM4-5) or FITC- anti-CD8α (53-6.7) and with biotin-labelled anti-CD25 (IL-2Rα) (7D4) followed by Streptavidin-phycoerythrin. Additionally, for the biotin-labelled antibodies FITC-labelled Streptavidin was used. FACS analysis of recovered cells was performed on the peak day of proliferation (day 4), as determined by [³H]thymidine incorporation.

For apoptosis assays, fresh MLN cells (MLNC) and splenic T cells (SPLTC) were isolated from BALB/c mice, 8-10 weeks old. MLNC comprising >90% CD3⁺ T cells, as determined by flow cytometric analysis, were incubated for 2 h in petri dishes (Costar, Cambridge, MA) in complete medium containing 10% FCS, at 37° C in 5% CO₂ and 95% air to remove adherent cells. The non-adherent fraction was subsequently pipetted off, pelleted and washed twice in HBSS before use. SPLTC were purified by negative selection using glass beads coated with normal mouse serum followed by rabbit anti-mouse γ-globulins as described (wigzell, H. 1976. Specific affinity fractionation of lymphocytes using glass or plastic bead columns. *Scand. J. Immunol*. 5:(suppl.5) 23.). The selected population of T cells were >90% CD3⁺ as determined by flow cytometric analysis.

CD4⁺ and CD8⁺ T cells were separated as follows: non-adherent MLNC were labelled with rat phycoerythrin-anti-mouse CD4 (4708-02) or FITC-anti-mouse CD8α (53-6.7) (PharMingen) and were then incubated with MACS colloidal super-paramagnetic microbeads conjugated with goat anti-rat IgG (H + L) F(ab')₂ (PharMingen), according to the manufacturer's instructions. These were applied to mini-MACS columns (Miltenyi Biotec, Bergisch Gladbach, Germany) in order to separate both positively (>99% pure) and negatively (>90% pure) selected populations of CD4 and CD8+ T cells, as determined by flow cytometric analysis.

Two methods were used for quantification of apoptosis: i) staining DNA with acridine orange to examine nuclear morphology and, ii) cell cycle analysis following staining DNA with propidium iodide and with either anti-CD4 or anti-CD8 antibodies. Cultures of 2x10⁶/ml MLNC, SPLTC and fractionated MLNC were established in complete medium containing 10% FCS, in the absence or presence of 80 µg/ml of either EtxB or EtxB(G33D) and examined from 4 to 18 h. Following incubation, cells were pelleted, washed with HBSS and stained with 5 µg/ml acridine orange (Sigma). Thymocytes were isolated and treated in the absence or in the presence of 10⁻⁷M dexamethasone and used as a positive control for cells undergoing apoptosis. Nuclear morphological changes in lymphocytes were examined by conventional or confocal fluorescence microscopy (Leica TCS 4D). The proportion of CD4⁺ and CD8⁺ SPLTC in the sub-G₀/G₁ stage of the cell cycle was determined by flow cytometric analysis of the DNA content following staining with propidium iodide as described (O'Connor, P.M., Jackman, J., Jondle, D., Bhatia, K., Magrath, I. and Kohn, K.W. 1993. Role of p53 tumor suppressor gene in cell cycle arrest and radiosensitivity of Burkitt's lymphoma cell lines. *Cancer.Res*. 53:4776.). Cells isolated from 18 h cultures of SPLTC incubated alone or with 40 µg/ml EtxB or EtxB(G33D) were stained with FITC rat anti-CD4 or FITC-anti-CD8α. Stained cells were adjusted to 1x10⁶/ml in cold HBSS containing 20mM HEPES and 0.5 mM EDTA and were fixed with cold ethanol added dropwise. Then, 50 µg/ml propidium iodide and 40 µg/ml ribonuclease A (DNase free) were added, and the cells incubated for 1 h at room temperature. The relative intensity of DNA staining with propidium iodide in CD4 and CD8⁺ T cells was determined by gating on cells co-stained with each mAB.

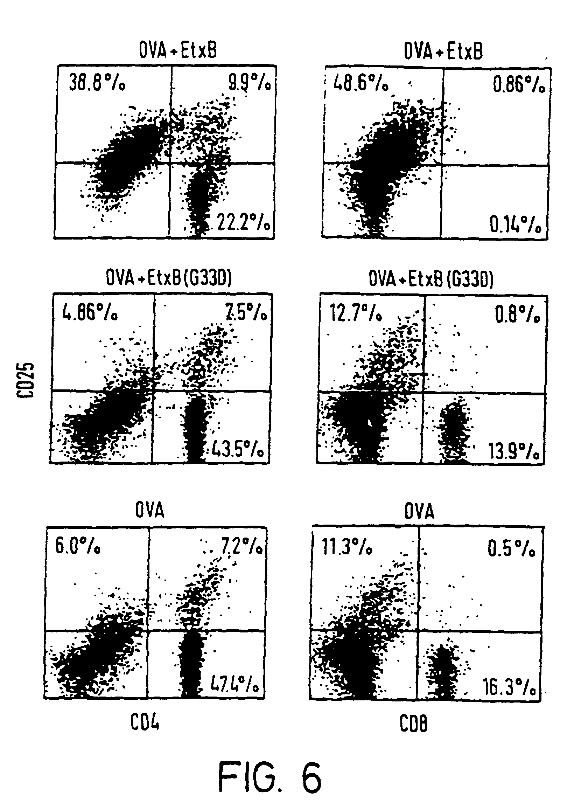

In Example 1, the observation that CD8⁺ T cells are completely depleted from cultures of lymph node cells proliferating in response to EtxB suggested that EtxB exerts a polyclonal effect on this T cell subset. To investigate whether such effects are dependent on the activation of EtxB responsive cells, cultures were established from OVA-primed mice and stimulated with OVA alone or with OVA plus either EtxB or the mutant EtxB(G33D). Similar peak levels of proliferation (day 4 of culture in each case) were achieved in the presence of OVA alone, OVA plus EtxB or OVA plus EtxB(G33D) (9734 ± 347, 12,031 ± 135 and 9305 ± 290 c.p.m. respectively). However, there was a dramatic difference in the distribution of T cell subsets in these cultures after 4 days (Figure 6). All cultures contained CD4⁺ T cells of which similar proportions co-expressed the activation marker CD25. However, CD8⁺ T cells were undetectable in cultures incubated with OVA plus EtxB, but were clearly present (although not activated as assessed by CD25 expression) in cultures with OVA plus EtxB(G33D) or OVA alone. This establishes that EtxB induces depletion of CD8⁺ T cells responding to an antigen other than EtxB. Moreover, the absence of such a response to EtxB(G33D) indicates that depletion is triggered following toxoid receptor interaction. It was also noted that the presence of wild-type EtxB caused a significant increase in the proportion of B cells of which a large number were CD25⁺ (not shown) as had previously been found for EtxB responsive cultures (Example 1). It is therefore concluded that receptor occupancy by EtxB exerts profound immunomodulatory effects on lymphocytes irrespective of their antigen specificity.

The possibility that CD8+ T cells undergo apoptosis when cultured in the presence of EtxB was investigated. MLNC or purified SPLTC, from unprimed mice, were incubated with EtxB or EtxB(G33D) and changes in cell nuclear morphology after staining with acridine orange were recorded over a period of 4 to 18 h (Table 3 and Figure 7). Cell morphological changes were characterized by the presence of condensation of chromatin resulting in the lobular appearence of the nucleus (Figure 7). Other cell features such as blebbing of the plasma membrane and the presence of apoptotic bodies were also observed. These morphological changes occurred in approximately one third of each of the cell preparations treated with EtxB, whereas a much lower incidence was observed in cells cultured with EtxB(G33D) or without exogenous antigen (Table 3). Since CD8⁺ T cells accounted for - 35-40% of the MLNC and SPLTC preparations, depletion of these cells could account for the observed apoptosis. To establish if this was the case, populations of purified CD8 and CD4⁺ T cells were cultured for 18 h in the presence of antigens (Table 3). Similar percentages of morphological changes were induced in negatively selected populations of CD4⁺ T cells (containing >90% CD4-bearing cells) on treatment with either EtxB, EtxB(G33D) or no antigen, indicating that binding of EtxB to its receptor does not trigger apoptosis in this T cell subset. In contrast, >70% of the negatively selected CD8⁺ T cells (>90% pure) exhibited morphological changes when cultured with wild-type EtxB; while incubation with either no antigen or EtxB(G33D) caused changes in only 11-19% of this T cell population, respectively. Further, the presence of low numbers of contaminating cells in the purified populations used (∼10% in each case) could not account for the observed effects since more highly purified populations containing >99% of CD8 or CD4⁺ T-cells (isolated by positive selection) responded to EtxB in a similar manner (60% were apoptotic in the presence of EtxB, compared with 7% for both no antigen and EtxB(G33D) treatments) (Table 3). Apoptosis was detected in 40% and 98% of thymocytes after 18 h incubation in the absence or in the presence of dexamethasone respectively.

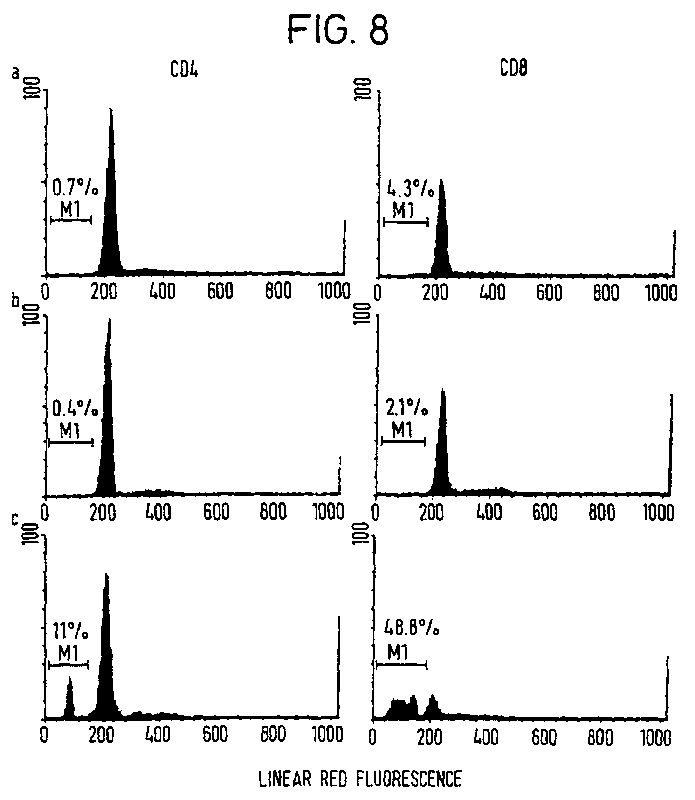

To demonstrate that the morphological changes observed in our cultures were consistent with the induction of apoptosis, the appearance of subdiploid DNA in cultures of SPLTC treated for 18 h with EtxB was evaluated. Cells were subjected to flow cytomeric analysis after co-staining with propidium iodide and either anti-CD8 or anti-CD4 antibodies (Figure 8). Approximately 48 % of the CD8⁺ T cells from cultures incubated with EtxB fell below the diploid G₀/G₁ peak of propidium iodide staining, indicating that they were undergoing apoptosis (O'Connor, P.M. et al, *supra*). A small proportion of cells expressing CD4, in cultures with EtxB, also exhibited sub-G₀/G₁ levels of DNA (∼11%; which may result from the death of such a high proportion of CD8⁺ T cells). In contrast, the majority of CD4 or CD8⁺ T cells cultured without antigen or in the presence of EtxB(G33D) were in G₀/G₁ phase of the cell cycle, with <5% exhibiting apoptosis. We conclude that the observed nuclear morphological changes, and the presence of sub-G₀/G₁ levels of DNA, in a substantial proportion of CD8⁺ T cells treated with EtxB demonstrate a selective apoptosis triggered by the cholera-like enterotoxoid. The failure of the receptor binding mutant, EtxB(G33D), to induce similar effects demonstrates that the induction of CD8⁺ T cell apoptosis is linked to its ability to bind to GM1 ganglioside.

### Example 3

Groups of 8 male DBA/1 mice were either unchallenged (group A) or were each injected with 100µg of bovine collagen in CFA on day 0 by intra-dermal (i.d.) injection into the flank. Collagen injected mice were either left unprotected (group B; positive control) or attempts were made to prevent disease development by the administration i.d. at an adjacent site to collagen challenge of; 100µg of EtxB in IFA on day 0 (group C), 100µg of EtxB in IFA on day 14 (group D), or 100µg EtxB(G33D) in IFA on day 0 (group E). All animals, except those in group A, received a boosting dose of collagen in IFA i.d. on day 21, and disease severity was assessed on day 45 by measuring hind limb ankle thickness (experiment A) or scoring each hind limb digit for swelling (scale 0-3 where 0 = normal, and 3 = maximal swelling; experiment B).

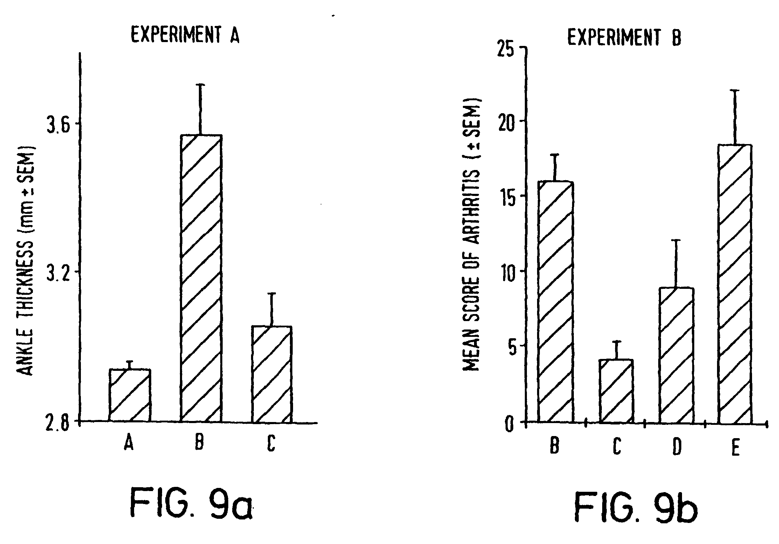

The results obtained are illustrated in Figures 9a and 9b. These show that EtxB, but not EtxB(G33D), dramatically protects mice from the development of collagen-induced arthritis.

### Example 4a

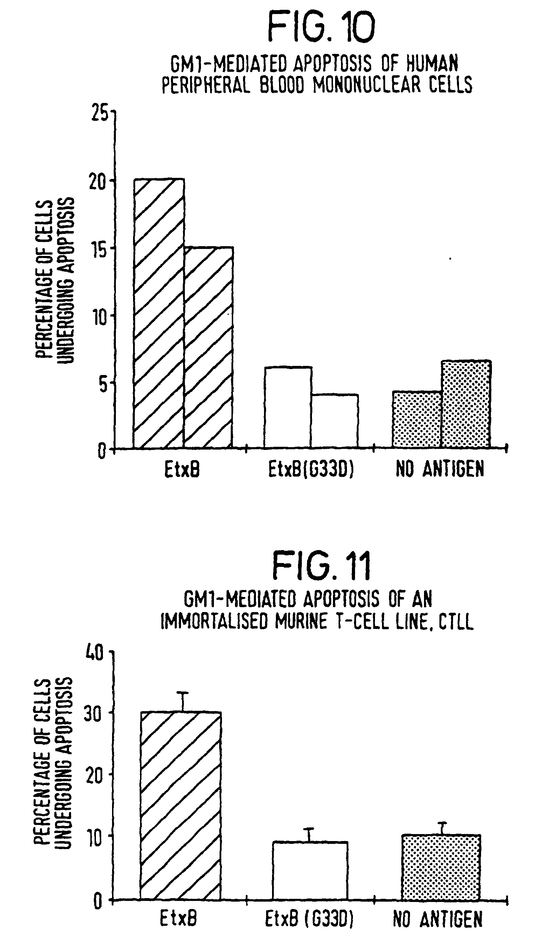

Two separate human buffy coat samples (obtained from normal human blood donors) were used as a source of mononuclear cells. Cells were isolated over Ficoll- paque and washed extensively before culture in the absence of antigen or with 80µg/ml of either EtxB or EtxB(G33D) as indicated. Prior to culture the cell populations comprised 24% CD8+, 27% CD4+ and 27% CD8+, 22.9% CD4+ for each sample respectively. After culture for 18 hours the appearance of apoptotic cells was assessed in samples of cells stained with acridine orange (as detailed under Example 2). The results obtained are shown in Figure 10; these illustrate that EtxB but not EtxB(G33D) induces apoptosis in a population of normal human peripheral blood mononuclear cells.

### Example 4b

The murine T cell line, CTLL-2, was cultured to confluence and then the cells washed before being reseeded at 1 x 10⁶ cells/ml in the absence of antigen or with 80µg/ml of either EtxB or EtxB(G33D) as indicated. After 18 hours samples were removed and the percentage of cells showing signs of apoptosis was assessed using acridine orange (detailed under Example 2). The results obtained are illustrated in Figure 11. They show that cross-linking of GM1 leads to apoptosis in a proportion of murine CTLL cells.

**Table 1 -**

| Lymphocyte proliferation in the presence of EtxB or EtxB (G33D) | | | | |
|---|---|---|---|---|
| Dose µg/ml | EtxB | EtxB (G33D) | EtxB* | EtxB (G33D) * |
| 0 | 117.9 | 146.8 | 124.5 | 116.1 |
| | (7.9) | (3.5) | (14.6) | (6.35) |
| 5 | 4928 | 2860 | 2424 | 1431.5 |
| | (98.7) | (3.8) | (88.3) | (37.5) |
| 10 | 6978 | 3681 | 2518 | 4231 |
| | (30.6) | (4.6) | (21.6) | (96.4) |
| 20 | 7084 | 6912 | 4394 | 5075 |
| | (100) | (47.3) | (42.1) | (24.8) |
| 40 | 8844 | 8586 | 7431 | 4368.5 |
| | (26) | (143.7) | (45.3) | (118.9) |
| 80 | 10246 | 12510 | 7986 | 7276 |
| | (30.7) | (121.8) | (210.3) | (369.5) |
| 160 | 11311 | 13525 | ----- | ----- |
| | (247) | (352.7) | | |

Mice were injected i.p. with 30µg of EtxB (G33D) in complete Freund's adjuvant (CFA). Mesenteric lymph nodes were isolated 10 days later. Cells were isolated and incubated for 4 days in the presence of EtxB, EtxB (G33D) or disassembled monomeric forms of these proteins (*), generated by heating at 95°C. Proliferation was determined by addition of 1µCi of (³H) dThd for the last 6 hours on day 4. Data represents mean cpm and SEM of triplicate wells. Cells isolated from unimmunized mice gave <1500 cpm (dose 160µg/ml).

**Table 2 -**

| Cytokine analysis in the presence of EtxB or EtxB (G33D) | | |
|---|---|---|
| Protein | IL-2 (pg/ml) | IFN-γ (pg/ml) |
| EtxB | 318 | 2700 |
| EtxB (G33D) | 67 | 4068 |

Mice were injected with EtxB (G33D) in CFA and mesenteric lymph nodes cells were isolated 10 days later. Cells were then incubated *in vitro* with either EtxB or EtxB(G33D) and samples of supernatants analysed for cytokine content on day 5 of cellular proliferation.

**Table 3 -**

| EtxB-receptor mediated apoptosis in fractionated lymphocyes. | | | | |
|---|---|---|---|---|
| Cells | Time (h) | No antigen | EtxB(G33D) | EtxB |
| MLNC | 4 | 0^{a} (8) | 2 (0) | 1 (3) |
| | 18 | 8 (10) | 5 (18) | 29 (35) |
| SPLTC | 4 | 3 (7) | 2 (6) | 3 (5) |
| | 18 | 17 (5) | 16.5 (12) | 31 (32) |
| Negative selection | | | | |
| CD4 | 18 | 5 (37) | 6 (31) | 9 (35) |
| CD8 | 18 | 18 (11) | 19 (15) | 76 (73) |
| Positive selection | | | | |
| CD4 | 18 | 6 | 4 | 6 |
| CD8 | 18 | 7 | 7 | 60 |

Nuclear morphological changes in fractionated CD4 and CD8⁺ T cells after 4 or 18 h incubation in the absence of antigen, or with 80 µg/ml of EtxB or EtxB(G33D) were examined by fluorescence microscopy following staining with acridine orange. Whole MLN were depleted of adherant cells. SPLTC were isolated by negative selection in glass beads column coated with mouse γ-globulins and rabbit anti-mouse as a secondary antibody. Fractionated SPLTC were obtained following labelling with rat phycoerythrin-anti-mouse CD4 or FITC-anti-mouse CD8α which were then incubated with MACS colloidal super-paramagnetic microbeads conjugated with goat anti-rat IgG (H + L) F(ab')₂. These were separated using mini-MACS columns to obtain both the positively (>99% pure) and negatively (>90% pure) selected fractions of CD4 and CD8⁺ T cells. Nuclear morphological changes were examined from 4 to 18 h in a random sample of 200 cells per treatment as described in the legend to Fig. 7. Maximum percentage of apoptotic cells occurred after 18 h. The data in brackets when indicated represent results from another separate experiment. Data for MLN and SPLTC are representative of a total of four experiments.
^{a}Percentage apoptotic cells

## Claims

1. The use of an agent in the manufacture of a medicament for preventing and/or treating an autoimmune disease or human T cell leukaemia, wherein:
(i) the agent is Etx (*E.coli* heat-labile enterotoxin), EtxB (*E.coli* heat-labile enterotoxin, subunit B), Ctx (cholera toxin) and/or CtxB (cholera toxin, subunit B)
(ii) if the agent is to be co-administered with an antigenic determinant, then the agent and antigenic determinant are not so linked to form a single active agent.

2. The use according to claim 1, for preventing and/or treating an autoimmune disease.

3. The use according to claim 2, for preventing and/or treating rheumatoid arthritis, multiple sclerosis or diabetes.

4. The use according to any preceding claim, wherein the agent is EtxB.

5. The use according to any preceding claim, wherein the agent is to be administered without coadministration of a self or cross-reacting antigen.

6. A pharmaceutical composition comprising an agent for use in the prevention and/or treatment of an autoimmune disease or human T cell leukaemia, wherein:
(i) the agent is Etx (*E.coli* heat-labile enterotoxin), EtxB (*E.coli* heat-labile enterotoxin, subunit B), Ctx (cholera toxin) and/or CtxB (cholera toxin, subunit B)
(ii) if the agent is to be co-administered with an antigenic determinant, then the agent and antigenic determinant are not so linked to form a single active agent.

7. A composition according to claim 6, for preventing and/or treating an autoimmune disease

8. A composition according to claim 6 or 7, for preventing and/or treating rheumatoid arthritis, multiple sclerosis or diabetes.

9. A composition according to any of claims 6 to 8, wherein the agent is EtxB.

10. A composition according to any of claims 6 to 9, wherein the agent is to be administered without coadministration of a self or cross-reacting antigen.

11. A kit comprising a pharmaceutical composition in accordance with any of claims 6 to 9 and separate therefrom a pharmaceutical composition comprising self- or cross-reacting antigenic determinant.

12. The use of an agent in the manufacture of a medicament for preventing and/or treating transplant rejection or GVHD, wherein:
(i) the agent is Etx (*E.coli* heat-labile enterotoxin) and/or EtxB (*E. coli* heat-labile enterotoxin, subunit B)
(ii) if the agent is to be co-administered with an antigenic determinant, then the agent and the antigenic determinant are not so linked to form a single active agent.

13. A pharmaceutical composition comprising an agent for use in the prevention and/or treatment of transplant rejection or GVHD, wherein:
(i) the agent is Etx (*E.coli* heat-labile enterotoxin) and/or EtxB (*E.coli* heat-labile enterotoxin, subunit B)
(ii) if the agent is to be co-administered with an antigenic determinant, then the agent and the antigenic determinant are not so linked to form a single active agent.

## Patentansprüche

1. Verwendung eines Mittels bei der Herstellung eines Medikaments zur Verhinderung und/oder Behandlung einer Autoimmunerkrankung oder humaner T-Zell-Leukämie, wobei
(i) das Mittel Etx (*E.coli* hitzelabiles Enterotoxin), EtxB (*E.coli* hitzelabiles Enterotoxin, Untereinheit B), Ctx (Choleratoxin) und/oder CtxB (Choleratoxin, Untereinheit B) ist,
(ii) wenn das Mittel gemeinsam mit einer antigenen Determinante zu verabreichen ist, dann sind das Mittel und die antigene Determinante nicht so miteinander verknüpft, daß sie ein einzelnes aktives Mittel bilden.

2. Verwendung nach Anspruch 1 zur Verhinderung und/oder Behandlung einer Autoimmunerkrankung.

3. Verwendung nach Anspruch 2 zur Verhinderung und/oder Behandlung von rheumatoider Arthritis, multipler Sklerose oder Diabetes.

4. Verwendung nach einem der vorangegangenen Ansprüche, wobei das Mittel EtxB ist.

5. Verwendung nach einem der vorangegangenen Ansprüche, wobei das Mittel ohne eine gemeinsame Verabreichung eines selbst- oder kreuzreagierenden Antigens zu verabreichen ist.

6. Pharmazeutische Zusammensetzung, enthaltend ein Mittel zur Verwendung bei der Verhinderung und/oder Behandlung einer Autoimmunerkrankung oder humaner T-Zell-Leukämie, wobei
(i) das Mittel Etx (*E.coli* hitzelabiles Enterotoxin), EtxB (*E.coli* hitzelabiles Enterotoxin, Untereinheit B), Ctx (Choleratoxin) und/oder CtxB (Choleratoxin, Untereinheit B) ist,
(ii) wenn das Mittel gemeinsam mit einer antigenen Determinante zu verabreichen ist, dann sind das Mittel und die antigene Determinante nicht so miteinander verknüpft, daß sie ein einzelnes aktives Mittel bilden.

7. Zusammensetzung nach Anspruch 6 zur Verhinderung und/oder Behandlung einer Autoimmunerkrankung.

8. Zusammensetzung nach Anspruch 6 oder 7 zur Verhinderung und/oder Behandlung von rheumatoider Arthritis, multipler Sklerose oder Diabetes.

9. Zusammensetzung nach einem der Ansprüche 6 bis 8, wobei das Mittel EtxB ist.

10. Zusammensetzung nach einem der Ansprüche 6 bis 9, wobei das Mittel ohne eine gemeinsame Verabreichung eines selbst- oder kreuzreagierenden Antigens zu verabreichen ist.

11. Kit, enthaltend eine pharmazeutische Zusammensetzung nach einem der Ansprüche 6 bis 9 und getrennt davon eine pharmazeutische Zusammensetzung, die eine selbst- oder kreuzreagierende antigene Determinante enthält.

12. Verwendung eines Mittels bei der Herstellung eines Medikaments zur Verhinderung und/oder Behandlung von Transplantatabstoßung oder GVHD, wobei
(i) das mittel Etx (*E.coli* hitzelabiles Enterotoxin) oder EtxB (*E.coli* hitzelabiles Enterotoxin, Untereinheit B) ist,
(ii) wenn das Mittel gemeinsam mit einer antigenen Determinante zu verabreichen ist, dann sind das Mittel und die antigene Determinante nicht so miteinander verknüpft, daß sie ein einzelnes aktives Mittel bilden.

13. Pharmazeutische Zusammensetzung, enthaltend ein Mittel zur Verwendung bei der Verhinderung und/oder Behandlung von Transplantatabstoßung oder GVHD, wobei
(i) das mittel Etx (*E.coli* hitzelabiles Enterotoxin) oder EtxB (*E.coli* hitzelabiles Enterotoxin, Untereinheit B) ist,
(ii) wenn das Mittel gemeinsam mit einer antigenen Determinante zu verabreichen ist, dann sind das Mittel und die antigene Determinante nicht so miteinander verknüpft, daß sie ein einzelnes aktives Mittel bilden.

## Revendications

1. Utilisation d'un agent dans la production d'un médicament pour la prévention et/ou le traitement d'une maladie auto-immune ou de la leucémie à lymphocytes T humaine, dans laquelle :
(i) l'agent est Etx (entérotoxine thermolabile de *E*. *coli*), EtxB (entérotoxine thermolabile de *E*. *coli*, sous-unité B), Ctx (toxine cholérique) et/ou CtxB (toxine cholérique, sous-unité B) ;
(ii) si l'agent est destiné à être co-administré avec un déterminant antigénique, alors l'agent et le déterminant antigénique ne sont pas liés de manière à former un seul agent actif.

2. Utilisation suivant la revendication 1, pour la prévention et/ou le traitement d'une maladie auto-immune.

3. Utilisation suivant la revendication 2, pour la prévention et/ou le traitement de la polyarthrite rhumatoïde, de la sclérose en plaques ou du diabète.

4. Utilisation suivant l'une quelconque des revendications précédentes, dans laquelle l'agent est EtxB.

5. Utilisation suivant l'une quelconque des revendications précédentes, dans laquelle l'agent est destiné à être administré sans co-administration d'un auto-antigène ou antigène à réaction croisée.

6. Composition pharmaceutique comprenant un agent destiné à être utilisé dans la prévention et/ou le traitement d'une maladie auto-immune ou de la leucémie à lymphocytes T humaine, dans laquelle :
(i) l'agent est Etx (entérotoxine thermolabile de *E. coli*), EtxB (entérotoxine thermolabile de *E. coli*, sous-unité B), Ctx (toxine cholérique) et/ou CtxB (toxine cholérique, sous-unité B) ;
(ii) si l'agent est destiné à être co-administré avec un déterminant antigénique, alors l'agent et le déterminant antigénique ne sont pas liés de manière à former un seul agent actif.

7. Composition suivant la revendication 6, pour la prévention et/ou le traitement d'une maladie auto-immune.

8. Composition suivant la revendication 6 ou 7, pour la prévention et/ou le traitement de la polyarthrite rhumatoïde, de la sclérose en plaques ou du diabète.

9. Composition suivant l'une quelconque des revendications 6 à 8, dans laquelle l'agent est EtxB.

10. Composition suivant l'une quelconque des revendications 6 à 9, dans laquelle l'agent est destiné à être administré sans co-administration d'un auto-antigène ou antigène à réaction croisée.

11. Kit comprenant une composition pharmaceutique suivant l'une quelconque des revendications 6 à 9 et, à l'état séparé de celle-ci, une composition pharmaceutique comprenant un déterminant auto-antigénique ou antigénique à réaction croisée.

12. Utilisation d'un agent dans la production d'un médicament destiné à la prévention et/ou au traitement du rejet d'un transplant ou de la réaction du greffon contre l'hôte (GVHD), dans laquelle :
(i) l'agent est Etx (entérotoxine thermolabile de *E. coli*) et/ou EtxB (entérotoxine thermolabile de *E. coli*, sous-unité B) ;
(ii) si l'agènt est destiné à être co-administré avec un déterminant antigénique, alors l'agent et le déterminant antigénique ne sont pas liés de manière à former un seul agent actif.

13. Composition pharmaceutique comprenant un agent destiné à être utilisé dans la prévention et/ou le traitement du rejet d'un transplant ou de la réaction du greffon contre l'hôte (GVHD), dans laquelle :
(i) l'agent est Etx (entérotoxine thermolabile de *E. coli*) et/ou EtxB (entérotoxine thermolabile de *E. coli*, sous-unité B) ;
(ii) si l'agent est destiné à être co-administré avec un déterminant antigénique, alors l'agent et le déterminant antigénique ne sont pas liés de manière à former un seul agent actif.
